# EUROPÄISCHE PATENTANMELDUNG

(11) **EP 3 547 254 A1**
(43) Veröffentlichungstag der Anmeldung: **02.10.2019**
(21) Anmeldenummer: 18165007.8
(22) Anmeldetag: 29.03.2018
(51) Int. Cl.: G06T 7/00, G06N 3/04

(54) **ANALYSE-VERFAHREN UND ANALYSEEINHEIT ZUR ERMITTLUNG RADIOLOGISCHER ERGEBNISDATEN**

(71) Anmelder: Siemens Healthcare GmbH, 91052 Erlangen (DE)
(72) Erfinder: Huber, Peter, 91575 Windsbach (DE); Krauß, Bernhard, 90559 Burgthann (DE); Schmidt, Bernhard, 90766 Fürth (DE); Schmidt, Sebastian, 91085 Weisendorf (DE)

(57) **Zusammenfassung**

Die Erfindung betrifft ein Analyse-Verfahren zur automatischen Ermittlung radiologischer Ergebnisdaten von Radiologie-Datensätzen (D1, D2, D3, D4), umfassend die Schritte:
- Bereitstellung von mindestens zwei Radiologie-Datensätzen (D1, D2, D3, D4) zumindest basierend auf Röntgendaten mit verschiedenen Röntgenenergiespektren (E1, E2, E3, E4),
- Bereitstellung einer Analyseeinheit (6) umfassend ein zu einer Analyse von Radiologie-Datensätzen (D1, D2, D3, D4) ausgebildetes Neuronales Netzwerk (NN), welches eine Eingangsschicht (N₀) mit mehreren Zellen (Z), Zwischenschichten (N₁) und eine Ausgangsschicht (Nᵢ) umfasst,
- Analyse der Radiologie-Datensätze (D1, D2, D3, D4) mittels des Neuronalen Netzwerks (NN), wobei den Zellen der Eingangsschicht (N₀) zur gemeinsamen Bearbeitung Teilmengen des ersten Radiologie-Datensatzes (D1) sowie zumindest des zweiten Radiologie-Datensatzes (D2, D3, D4) zugeordnet werden,
- Erfassung von Ergebnisdaten an der Ausgangsschicht.

Die Erfindung betrifft des Weiteren eine entsprechende Analyseeinheit bzw. ein Neuronales Netzwerk, sowie eine entsprechende Steuereinrichtung zur Steuerung eines Röntgenaufnahmesystems, eine entsprechende Befundungsstation, und ein entsprechendes medizintechnisches bildgebendes System.

## Beschreibung

Die Erfindung betrifft ein Analyse-Verfahren und eine Analyseeinheit bzw. ein Neuronales Netzwerk zur automatischen Ermittlung radiologischer Ergebnisdaten von Radiologie-Datensätzen, insbesondere spektrale Computertomographiedaten, sowie eine entsprechende Steuereinrichtung zur Steuerung eines Röntgenaufnahmesystems, eine entsprechende Befundungsstation zur Kopplung mit einem Röntgenaufnahmesystem, und ein entsprechendes medizintechnisches bildgebendes System.

In der radiologischen Bildgebung ist eine effektive Analyse und Weiterverarbeitung von radiologischen Aufnahmen essentiell. Dazu werden in zunehmendem Maße Verfahren basierend auf maschinellem Lernen verwendet. Mit solchen Verfahren können Objekte in Bildern, z.B. krankhafte Veränderungen, automatisch erkannt und dem befundenden Arzt angezeigt werden.

Oft verwendete Verfahren basieren auf Neuronalen Netzen, bzw. basieren solche Verfahren auf simulierten Neuronen. Beispiele für solche Neuronalen Netze sind tiefe Neuronale Netze (engl.: "Deep Learning") oder Faltungsnetzwerke (engl.: Convolutional Neural Networks "CNN"). Neuronale Netze umfassen in der Regel eine Eingangsschicht (erste Schicht), in welche die zu verarbeitenden Daten eingegeben werden, eine Anzahl von Zwischenschichten, welche der Verarbeitung dienen und eine Ausgangsschicht, die das Ergebnis der Bearbeitung bereitstellt.

Beispielsweise werden dabei in der Eingangsschicht des Netzwerks die Pixel (bei einer zweidimensionalen Messung), bzw. Voxel (bei einer dreidimensionalen Messung) den Zellen Der Schicht ("Neuronen") zugeordnet, so dass jedes Neuron den Signalwert des zugeordneten Pixels als Eingangsgröße erhält.

In der Ausgabeschicht sind dann so viele Neuronen enthalten, wie verschiedene Ergebnisse der Klassifikation möglich sind.

Zunehmend werden im Rahmen der Radiologietechnik, insbesondere im Hinblick auf die Computertomographie ("CT"), Aufnahmeverfahren eingesetzt, die mehrere Aufnahmeenergien nutzen. Seit neuerem werden in einigen Geräten sogar spektrale Detektoren verwendet, die nicht mehr nur einen Signalwert pro Pixel erzeugen, sondern auch die spektrale Energie der eintreffenden Photonen unterscheiden können.

Dieses Aufnahmeverfahren mit mehreren Aufnahmeenergien bzw. einem Energiespektrum wird jedoch derzeit nicht optimal ausgenutzt, was in Anbetracht des Potentials solcher Aufnahmen einen gravierenden Nachteil darstellt. Aus spektralen CT-Daten werden in der Regel herkömmliche CT-Bilder mit nur einem Signalwert pro Pixel rekonstruiert, wobei die verschiedenen spektralen Anteile unterschiedlich gewichtet werden, um einen besonderen Bildeindruck zu erzeugen (z.B. Hervorhebung eines Kontrastmittels, welches ein spezifisches Absorptionsspektrum besitzt).

Es ist eine Aufgabe der vorliegenden Erfindung, ein alternatives, komfortableres Analyse-Verfahren, eine Analyseeinheit bzw. ein Neuronales Netzwerk zur automatischen Ermittlung radiologischer Ergebnisdaten von Radiologie-Datensätzen, sowie eine entsprechende Steuereinrichtung zur Steuerung eines Röntgenaufnahmesystems, eine entsprechende Befundungsstation zur Kopplung mit einem Röntgenaufnahmesystem, und ein entsprechendes medizintechnisches bildgebendes System zur Verfügung zu stellen, mit denen die oben beschriebenen Nachteile vermieden werden können.

Diese Aufgabe wird durch ein Analyse-Verfahren gemäß Patentanspruch 1, eine Analyseeinheit gemäß Patentanspruch 8, ein Neuronales Netzwerk gemäß Patentanspruch 9, eine Steuereinrichtung gemäß Patentanspruch 10, eine Befundungsstation gemäß Patentanspruch 11 und ein medizintechnisches bildgebendes System gemäß Patentanspruch 12 gelöst.

Das erfindungsgemäße Analyse-Verfahren bzw. die erfindungsgemäße Analyseeinheit dient zur automatischen Ermittlung radiologischer Ergebnisdaten auf Basis von bzw. aus Radiologie-Datensätzen.

Die Radiologie-Datensätze sind bevorzugt CT-Datensätze also mittels einen Computertomographiesystem erzeugt. Sie können aber auch auf einem anderen Gerät erzeugt werden, welches mit Röntgenstrahlung arbeitet, z. B. einem einfachen Röntgendurchleuchtungsgerät, einem Mammographiegerät, einem Angiographen etc.

Unter einem Radiologie-Datensatz kann im Folgenden zum einen der unmittelbar am Gerät, z.B. dem Computertomographiesystem, gemessene Rohdatensatz verstanden werden. Ebenso kann hierunter aber auch ein Bilddatensatz verstanden werden, der auf Basis dieser Rohdatensatzes rekonstruiert wurde. Dies hängt lediglich davon ab, ob im neuronalen Netzwerk unmittelbar mit den Rohdaten gearbeitet werden soll oder mit den Bilddaten.

Diese Radiologie-Datensätze repräsentieren dabei unterschiedliche Röntgenaufnahmen mit mehreren Röntgenenergiespektren. Es ist dabei zu beachten, dass in der Realität bei einer Röntgenaufnahme stets eine gewisse Energiebreite vorliegt, selbst wenn das "Röntgenenergiespektrum" schmalbandig ist. In der Regel wird die Aufnahme jedoch mit einem Wert für eine diskrete Röntgenenergie bezeichnet, z. B. mit der eigestellten Spannung an der Röntgenquelle.

Das Röntgenenergiespektrum wird z.B. durch die Einstellung der Röntgenquelle bzw. des Detektors oder durch eine Auswahl von Messdaten des Detektors bestimmt, wenn dieser energieselektiv arbeiten kann. Soweit von "Röntgendaten eines Röntgenenergiespektrums" die Rede ist, werden damit also Messdaten eines Röntgenaufnahmesystems, beispielsweise eines Computertomographiesystems, verstanden, die mit dem entsprechenden Röntgenenergiespektrum erfolgt sind.

Bevorzugt sind hierbei "spektrale" Radiologie-Datensätze, die mit einem Röntgenaufnahmesystem aufgenommen wurden, das einen Röntgenstrahl mit einem breiten Röntgenenergiespektrum aussendet, wobei das Detektorsystem so gestaltet ist, dass es nicht mehr nur einen Signalwert pro Pixel erzeugt, sondern auch die spektrale Energie der eintreffenden Photonen unterscheiden können also energieselektiv sind. Spektrale Radiologie-Datensätze können also an Stelle eines einzigen Grauwertes pro Bildpunkt bzw. Messpunkt viele Werte aufweisen, die Aufnahmewerte über ein ausgedehntes Spektrum wiedergeben. Prinzipiell sind solche spektralen Radiologie-Datensätzen aber auch durch eine Zusammenfassung von Röntgendaten unterschiedlicher Röntgenenergiespektren erzeugbar, unabhängig davon, ob die Röntgendaten der unterschiedlichen Röntgenenergiespektren in einem Messvorgang, zum Beispiel mit einem energieselektiven Detektor, aufgenommen wurden oder zeitlich nacheinander.

Das erfindungsgemäße Analyse-Verfahren umfasst die Schritte:

### - Bereitstellung eines ersten Radiologie-Datensatzes

Der erste Radiologie-Datensatz basiert zumindest auf Röntgendaten eines ersten Röntgenenergiespektrums. Der erste Radiologie-Datensatz ist also bei der ersten Röntgenenergie aufgenommen worden.

### - Bereitstellung zumindest eines zweiten Radiologie-Datensatzes

Der zweite Radiologie-Datensatz basiert zumindest auf Röntgendaten eines zweiten Röntgenenergiespektrums, welches sich vom ersten Röntgenenergiespektrum unterscheidet. Dabei ist es aber nicht unbedingt erforderlich, dass die beiden Röntgenenergiespektren immer völlig disjunkt sein müssen auch wenn dies für die meisten Anwendungen durchaus vorteilhaft ist. Es kann dennoch für einige spezielle Anwendungen von Vorteil sein, wenn sich das erste und das zweite Röntgenenergiespektrum überlappen oder das eine Röntgenenergiespektrum im anderen Röntgenenergiespektrum enthalten ist.

Der Begriff "zumindest" besagt, dass noch weitere Radiologie-Datensätze bereitgestellt werden können, die mit weiteren Röntgenenergiespektren aufgenommen worden sind, wobei für diese weiteren Röntgenenergiespektren dasselbe gilt, wie zum zweiten Röntgenenergiespektrum: Die verschiedenen Röntgenenergiespektren sollten nicht identisch miteinander sein, können aber ggf. Teile der anderen Röntgenenergiespektren umfassen. Z.B. kann bei mehr als zwei Radiologie-Datensätzen einer der Radiologie-Datensätze auch die (ggf. normierte) Summe einer Anzahl der anderen Radiologie-Datensätze darstellen.

Bevorzugt ist das Verfahren dazu ausgelegt, mit mindestens drei, vier, fünf oder sechs Radiologie-Datensätzen zu arbeiten.

### - Bereitstellung einer Analyseeinheit

Diese Analyseeinheit umfasst ein zu einer Analyse von Radiologie-Datensätzen ausgebildetes, insbesondere schon (vor-)trainiertes, Neuronales Netzwerk, welches eine Eingangsschicht, eine Anzahl von Zwischenschichten und eine Ausgangsschicht umfasst. Die Eingangsschicht weist dabei mehrere Zellen auf, die bei vielen Neuronalen Netzen auch als "Neuronen" bezeichnet werden. Die Ausgangsschicht repräsentiert ein radiologisches Ergebnis, z.B. eine Reihe von möglichen Befunden, bevorzugt zusammen mit der Wahrscheinlichkeit, dass diese Befunde in den Aufnahmen vorliegen.

### - Analyse

In diesem Schritt erfolgt die Analyse des ersten Radiologie-Datensatzes und zumindest des zweiten Radiologie-Datensatzes mittels des Neuronalen Netzwerks. Dazu werden den Zellen der Eingangsschicht zur gemeinsamen Bearbeitung Teilmengen des ersten Radiologie-Datensatzes sowie zumindest des zweiten Radiologie-Datensatzes zugeordnet. Diese Teilmengen sind bevorzugt eine Gruppe von Bildpunktwerten (z.B. ein einziger oder mehrere Pixel bzw. Voxel), eine Gruppe von Rohdatenwerten (womit auch ein einziger Rohdatenwert gemeint sein kann) bzw. eine Menge der Bildinformationen. Das Neuronale Netzwerk ist dabei dazu ausgebildet, aus den Daten in der Eingangsschicht Ergebnisdaten in der Ausgangsschicht zu erzeugen.

Erfindungsgemäß wird also eine spezielle Zuordnung der Zellen der Eingangsschicht (Input Layer) zu den Eingangsdaten, bzw. dem Detektor des Röntgenaufnahmesystems (Messeinrichtung und Signalverarbeitung) vorgeschlagen. Die Verarbeitungsschichten (Zwischenschichten oder "Hidden Layers") zwischen Eingangsschicht und Ausgangsschicht können im Prinzip beliebig gewählt werden, es werden aber bevorzugt "tiefe" Netze verwendet, d.h. mit mehr als einer Zwischenschicht. Die Ausgangsschicht entspricht radiologischen "Eigenschaften", die den Bildinformationen - oder gegebenenfalls auch den Rohdaten direkt - entnehmbar sind. Das kann z.B. eine Diagnose sein (Lungenkrebs: Ja/Nein), morphologische Eigenschaften (Vorhandensein von Lungenknötchen) oder quantitative Eigenschaften (Volumen von Lungenknötchen).

Durch das Training des Neuronalen Netzwerks werden die Gewichtungen der Verbindungen in der Architektur des Netzwerks modifiziert. Üblicherweise werden dabei neuronale Netze so trainiert, dass Paare von Eingangsdaten und (valide) hierzu passende Ergebnisdaten verwendet werden. Im vorliegenden Fall wären das Paare von spektralen Radiologie-Datensätzen (z.B. CT-Datensätzen) und radiologischen Ergebnissen, z.B. Befunde, Diagnosen oder Messungen, welche händisch von Fachleuten oder auch automatisiert mit anderen Verfahren erstellt worden sind. Es können auch synthetische Paare verwendet werden, indem z.B. in Radiologie-Datensätzen zufällig Knötchen mit bekannten Durchmesser erzeugt werden und diese dann für das Training verwendet werden. Das Training kann dann mit bekannten Verfahren erfolgen, z.B. Rückpropagation.

Es wird an dieser Stelle darauf hingewiesen, dass auch im Rahmen der Analyse das Neuronale Netzwerk immer weiter trainiert und somit verbessert werden kann, indem beispielsweise die Ergebnisdaten verifiziert werden.

### - Erfassung von Ergebnisdaten

Nach der Bearbeitung der Radiologie-Datensätze durch das Neuronale Netzwerk liegen die Ergebnisdaten der Verarbeitung wie erwähnt an der Ausgangsschicht vor und können dort erfasst werden.

Eine erfindungsgemäße Analyseeinheit umfasst dementsprechend:
- Eine Datenschnittstelle zur Erfassung von mindestens zwei Radiologie-Datensätzen. Der erste Radiologie-Datensatz basiert, wie oben bereits ausführlich ausgeführt wurde, zumindest auf Röntgendaten eines ersten Röntgenenergiespektrums, der zweite Radiologie-Datensatz basiert zumindest auf Röntgendaten eines zweiten Röntgenenergiespektrums. Es können wie erwähnt noch weitere Radiologie-Datensätze erfasst werden.
- Ein zu einer Analyse von Radiologie-Datensätzen ausgebildetes Neuronales Netzwerk, welches eine Eingangsschicht, eine Anzahl von Zwischenschichten und eine Ausgangsschicht umfasst. Zu den Schichten gilt das oben ausgeführte.

Die erfindungsgemäße Analyseeinheit ist dabei zu einer Analyse des ersten Radiologie-Datensatzes und zumindest des zweiten Radiologie-Datensatzes (und ggf. weiterer Radiologie-Datensätze) mittels des Neuronalen Netzwerks ausgelegt. Dazu werden den Zellen der Eingangsschicht zur gemeinsamen Bearbeitung Teilmengen (s.o.) des ersten Radiologie-Datensatzes sowie zumindest des zweiten Radiologie-Datensatzes zugeordnet.

Zudem ist die Analyseeinheit zu einer Erfassung von Ergebnisdaten an der Ausgangsschicht ausgelegt.

Ein erfindungsgemäßes Neuronales Netzwerk umfasst eine Eingangsschicht mit mehreren Zellen, eine Anzahl von Zwischenschichten und eine Ausgangsschicht, welche ein radiologisches Ergebnis repräsentiert. Es ist dazu ausgelegt, dass den Zellen der Eingangsschicht zur gemeinsamen Bearbeitung Teilmengen eines ersten Radiologie-Datensatzes zumindest basierend auf Röntgendaten eines ersten Röntgenenergiespektrums sowie zumindest eines zweiten Radiologie-Datensatzes zumindest basierend auf Röntgendaten eines zweiten Röntgenenergiespektrums zugeordnet werden. Zudem ist das Neuronale Netzwerk dazu ausgelegt, radiologische Ergebnisdaten zu generieren und an der Ausgangsschicht zur Verfügung zu stellen.

Eine erfindungsgemäße Steuereinrichtung zur Steuerung eines Röntgenaufnahmesystems, insbesondere eines Computertomographiesystems umfasst eine erfindungsgemäße Analyseeinheit bzw. ist sie zur Durchführung eines erfindungsgemäßen Analyse-Verfahrens ausgestaltet.

Eine erfindungsgemäße Befundungsstation zur Kopplung mit einem Röntgenaufnahmesystem ausgelegt, insbesondere mit einem Computertomographiesystem. Die Befundungsstation umfasst eine erfindungsgemäße Analyseeinheit bzw. ist sie zur Durchführung eines erfindungsgemäßen Analyse-Verfahrens ausgestaltet. Die Befundungsstation kann in einem Röntgenaufnahmesystem integriert sein, z.B. in dessen Steuereinrichtung. In der Praxis wird es jedoch häufiger der Fall sein, dass sie in Form einer leistungsfähigen Rechenvorrichtung im Rahmen eines Radiologieinformationssystems ("RIS") oder eines Bildarchivierungs- und Kommunikationssystems (Picture Archiving and Communication System, "PACS") vorliegt.

Ein erfindungsgemäßes medizintechnisches bildgebendes System umfasst ein Röntgenaufnahmesystem und eine erfindungsgemäße Analyseeinheit. Die Analyseeinheit liegt in dem medizintechnischen bildgebenden System bevorzugt in Form einer erfindungsgemäßen Befundungsstation und/oder einer erfindungsgemäßen Steuereinrichtung vor. Die Steuereinrichtung kann dabei auch Teil des Röntgenaufnahmesystems sein.

Ein Großteil der zuvor genannten Komponenten, welche zu der Erfindung notwendig sind, können ganz oder teilweise in Form von Softwaremodulen in einem Prozessor einer entsprechenden Vorrichtung bzw. Steuereinrichtung realisiert werden. Eine weitgehend softwaremäßige Realisierung hat den Vorteil, dass auch schon bisher verwendete Vorrichtungen bzw. Steuereinrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in ein Rechensystem bzw. eine Speichereinrichtung einer Steuereinrichtung eines Röntgenaufnahmesystems ladbar ist, mit Programmabschnitten, um alle Schritte des erfindungsgemäßen Verfahrens auszuführen, wenn das Programm in dem Rechensystem bzw. der Steuereinrichtung ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen

Zum Transport zum Rechensystem bzw. zur Steuereinrichtung und/oder zur Speicherung an oder in dem Rechensystem bzw. der Steuereinrichtung kann ein computerlesbares Medium, z.B. ein Memorystick, eine Festplatte oder ein sonstiger transportabler oder fest eingebauter Datenträger dienen, auf welchem die von einem Rechensystem bzw. einer Rechnereinheit der Steuereinrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Bevorzugt wird zur Analyse einer Anzahl von Zellen der Eingangsschicht, insbesondere jeder Zelle, jeweils genau ein Bildpunktwert oder Rohdatenwert des ersten Radiologie-Datensatzes oder des zweiten Radiologie-Datensatzes zugeordnet. Diesen Zellen wird also kein großer Datenblock übergegeben, sondern lediglich der Wert eines Bildpunkts (Pixels oder Voxels) bzw. ein Datenwort.

Dabei wird bevorzugt jeder einzelne Bildpunktwert und/oder jeder einzelner Rohdatenwert der Radiologie-Datensätze einer Zelle der Eingangsschicht zugeordnet. Die jeweiligen Aufnahmen, die durch die Radiologie-Datensätze repräsentiert werden, werden also komplett an das Netzwerk übergeben.

Es können grundsätzlich zweidimensionale, dreidimensionale oder vierdimensionale Radiologie-Datensätze verwendet werden, wobei vierdimensionale Radiologie-Datensätze mehrere dreidimensionale Radiologie-Datensätze desselben Volumens umfassen, welche zu unterschiedlichen Zeiten gewonnen worden sind. Das entstehende Array hat also mit dem Wert n für die Anzahl der Röntgenenergiespektren, den Koordinaten x, y und z und der Zeit t das Format [n,x,y,z,t], wobei n,x,y immer vorhanden sein müssen. Die Anteile z und t können auch entfallen, wenn z.B. nur zweidimensionale Fälle behandelt werden.

Liegen beispielsweise n Aufnahmen als zweidimensionale Bilder mit x·y Pixeln vor und wird jeder Zelle ein Pixel zugeordnet, wobei die n Aufnahmen mit ihrer gesamten Bildinformation durch das Neuronale Netz verarbeitet werden, so enthält die Eingangsschicht dann n·x·y Neuronen. Die Zahl n ist dann auch die Anzahl der spektralen Gruppen, da die Aufnahmen alle eine nichtidentische Aufnahmeenergien repräsentieren. Die Werte x und y sind auch die Größe der Bildmatrix der rekonstruierten Bilder in x- und y-Richtung. In einem Beispielfall, in dem dreidimensionale Daten von Volumenaufnahmen mit dem Format x·y·z verarbeitet werden (und nicht als einzelne Schichtbilder) ergibt sich für die Anzahl der Zellen in der Eingangsschicht zur Verarbeitung der Voxel entsprechend n·x·y·z. Analog erfolgt eine Verarbeitung bzw. Zuordnung der Pixel bei drei- und vierdimensionalen Messungen bzw. Radiologiedatensätzen.

Bei einer bevorzugten Variante kann das Verfahren mehrstufig aufgebaut sein. Dabei wird in einem Analyseschritt nur ein Teil der zu analysierenden Radiologie-Datensätze analysiert. Ein anderer Teil der zu analysierenden Radiologie-Datensätze wird dann in mindestens einem weiteren Analyseschritt analysiert, der bevorzugt analog zu dem vorangehenden arbeitet. Die Radiologie-Datensätze werden also z.B. segmentweise analysiert: Teilbild für Teilbild bzw. werden nacheinander Teilbereiche der Rohdaten analysiert. Es ist mit dieser Ausführungsform möglich, das Neuronale Netzwerk als "Fenster" (engl.: "Sliding Window") über eine Anzahl von Radiologie-Datensätzen, insbesondere Bilddaten bzw. Bildaufnahmen, zu führen, was z.B. bei sehr großen Datenmengen und einer Analyse im Hinblick auf kleine Strukturen eine vorteilhafte Reduktion des Rechenaufwandes darstellen kann. Beispielsweise kann diesbezüglich ein relativ kleines Neuronales Netz (z.B. mit einem Raster von 20·20) über die Radiologie-Datensätze "fahren".

Bevorzugt umfasst das Neuronale Netzwerk mindestens 2, bevorzugt mindestens 5, insbesondere mindestens 20, Zwischenschichten. Dies hat den Vorteil, dass Prinzipien des "Deep Learning" eingesetzt werden können.

Grundsätzlich ist die Erfindung aber nicht auf eine bestimmte Art Netzwerk beschränkt. Bevorzugte Neuronale Netze sind Faltungsnetzwerke (engl.: Convolutional Neural Network, "CNN") oder ein Long-Term-Short-Term Memory-Netz ("LTSM"). LTSM-Netze sind dabei besonders von Vorteil, wenn zeitaufgelöste (vierdimensionale) Daten verwendet werden sollen.

Bevorzugt basiert zumindest einer der bereitgestellten Radiologie-Datensätze zumindest auf Röntgendaten zweier unterschiedlicher Röntgenenergiespektren beruht, vorzugsweise auf Kombinationsdaten von Radiologie-Datensätzen basierend auf Röntgendaten der unterschiedlichen Röntgenenergiespektren. Die betreffenden Daten sind dabei bevorzugt in Form einer (insbesondere gewichteten) Summe und/oder einer Differenz und/oder eines Quotienten und/oder eines Produkts miteinander verknüpft, wobei auch eine Kombination dieser Rechenoperationen bevorzugt ist, z.B. eine Summe zusammen mit einer Normierung.

Bevorzugt wird zur Bereitstellung des ersten Radiologie-Datensatzes und des zweiten Radiologie-Datensatzes ein Gesamt-Röntgen-Datensatz aufgeteilt. Dieser Gesamt-Röntgen-Datensatz wurde dabei mit einem dritten Röntgenenergiespektrum gemessen, welches das erste Röntgenenergiespektrum und das zweite Röntgenenergiespektrum umfasst. Vorzugsweise wird dies durch Aufteilung von Rohdaten einer Messung mit dem dritten Röntgenenergiespektrum in unterschiedliche Rohdatensätze und einer Rekonstruktion von unterschiedlichen Bilddatensätzen auf Basis der unterschiedliche Rohdatensätze erreicht. Der Gesamt-Röntgen-Datensatz kann dabei z.B.(ggf. ausschließlich) aus Rohdaten gebildet werden oder einen bereits rekonstruierten Bilddatensatz umfassten, je nachdem ob bei der Analyse direkt auf Basis der Rohdaten gearbeitet werden soll oder auf Basis von Bilddaten. Eine bevorzugte Analyseeinheit umfasst dazu bevorzugt eine Aufteilungseinheit welche zu einer solchen Aufteilung ausgelegt ist.

Bei einem bevorzugten medizintechnischen bildgebenden System, umfasst das Röntgenaufnahmesystem einen energieauflösenden Detektor, besonders bevorzugt einen direkt konvertierenden Detektor.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
Figur 1 einen Ablaufplan für einen möglichen Ablauf eines erfindungsgemäßen Analyse-Verfahrens,
Figur 2 eine Darstellung eines Ausführungsbeispiels zur Aufteilung und Analyse von Radiologie-Datensätzen,
Figur 3 eine Darstellung eines weiteren Ausführungsbeispiels zur Aufteilung und Analyse der Radiologie-Datensätzen,
Figur 4 eine grob schematische Darstellung eines Röntgenaufnahmesystems mit einem Ausführungsbeispiel einer erfindungsgemäßen Steuereinrichtung und Befundungsstation zur Durchführung des Verfahrens.

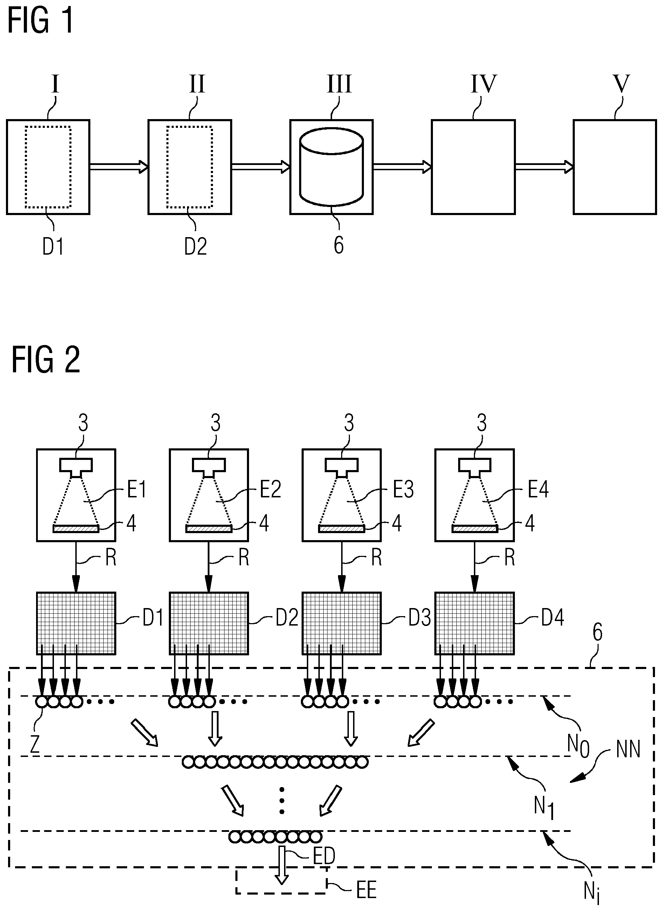

Figur 1 zeigt einen Ablaufplan für einen möglichen Ablauf eines erfindungsgemäßen Analyse-Verfahrens zur automatischen Ermittlung radiologischer Ergebnisdaten auf Basis von Radiologie-Datensätzen D1, D2.

In Schritt I erfolgt eine Bereitstellung eines ersten Radiologie-Datensatzes D1 basierend auf Röntgendaten eines ersten Röntgenenergiespektrums E1.

In Schritt II erfolgt eine Bereitstellung eines zweiten Radiologie-Datensatzes D2 basierend auf Röntgendaten eines zweiten Röntgenenergiespektrums E2. Die Bereitstellung des zweiten Radiologie-Datensatzes D2 (und ggf. weiterer Radiologie-Datensätze D3, D4 kann durchaus auch gleichzeitig mit dem ersten Radiologie-Datensatz D1 erfolgen.

In Schritt III erfolgt eine Bereitstellung einer erfindungsgemäßen Analyseeinheit 6.

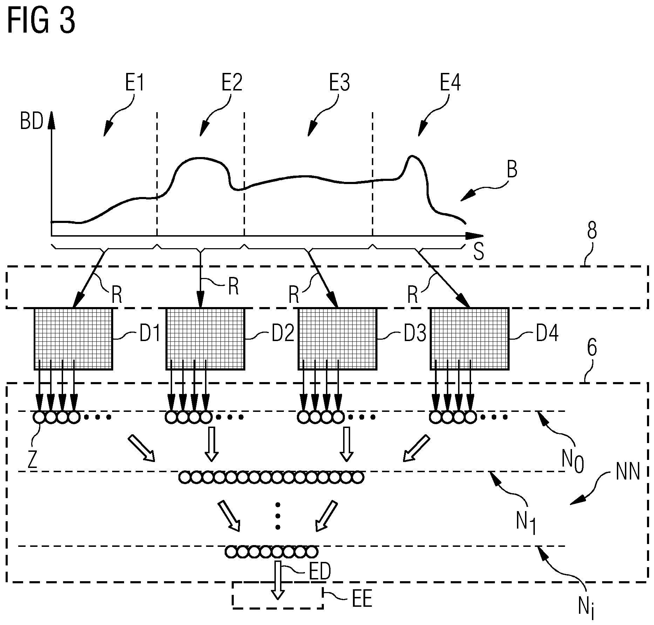

In Schritt IV erfolgt eine Analyse des ersten Radiologie-Datensatzes D1 und des zweiten Radiologie-Datensatzes D2 mittels der Analyseeinheit 6. Wie dies bevorzugt geschieht, wird in den Figuren 2 und 3 genauer dargestellt.

In Schritt V erfolgt eine Erfassung von Ergebnisdaten.

Im Folgenden wird wegen der besseren Verständlichkeit als Beispiel davon ausgegangen, dass mit Bilddaten gearbeitet wird, wobei aber ebenso Rohdaten anstelle der Bilddaten verwendet werden könnten, d.h. es kann dann auf den Schritt der Rekonstruktion verzichtet werden.

Ebenso wird im Folgenden davon ausgegangen, dass es sich bei den Messungen um computertomographische Aufnahmen handelt, wobei aber wie erwähnt die Erfindung nicht auf die Computertomographie beschränkt ist.

Figur 2 zeigt eine Darstellung eines Ausführungsbeispiels zur Analyse von - hier als Beispiel vier - Radiologie-Datensätzen D1, D2, D3, D4. Zuerst erfolgen in diesem Beispiel vier "getrennte" Messungen bzw. Aufnahmen mittels einer Röntgenquelle 3 und eines Detektors 4 bei jeweils unterschiedlichen Röntgenenergiespektren (E1, E2, E3, E4). Diese Aufnahmen können gleichzeitig, z.B. mit vier Paaren von Röntgenquellen 3 und Detektoren 4, oder nacheinander (ggf. mit einer einzigen Röntgenquelle, die mit unterschiedlichen Beschleunigungsspannungen betrieben wird) angefertigt werden.

Diese Messungen bzw. Aufnahmen ergeben vier Radiologie-Datensätze D1, D2, D3, D4.

In diesem Beispiel liegen wie erwähnt die Radiologie-Datensätze D1, D2, D3, D4 aus Gründen der besseren Anschaulichkeit in Form von zweidimensionalen Bildern mit einzelnen Pixeln vor, die aus Rohdaten der einzelnen Aufnahmen in einem Rekonstruktionsverfahren R rekonstruiert worden sind. Das Format dieser Bilder könnte beispielsweise mit einer Breite von x-Pixeln und einer Höhe von y-Pixeln angenommen werden.

Dabei ist es auch möglich, dass einer der Radiologie-Datensätze D1, D2, D3, D4 (oder mehrere) Daten aus zwei oder mehr kombinierten Aufnahmen mit unterschiedlichen Röntgenenergiespektren (E1, E2, E3, E4 umfasst).

Nun werden die Radiologie-Datensätze D1, D2, D3, D4 gemäß dem erfindungsgemäßen Verfahren zur Bearbeitung durch die Analyseeinheit 6 bereitgestellt. Diese umfasst ein zu einer Analyse von Radiologie-Datensätzen, hier also in Form von Bildaufnahmen B, ausgebildetes Neuronales Netzwerk NN, welches eine Eingangsschicht N₀ mit mehreren Zellen Z, eine Anzahl von Zwischenschichten N₁ und eine Ausgangsschicht Nᵢ umfasst, welche ein radiologisches Ergebnis repräsentiert. Die Anzahl der Zwischenschichten wäre hier i-1 mit i einer bevorzugten Anzahl zwischen 5 und 20.

Zur Analyse der Radiologie-Datensätze D1, D2, D3, D4 mittels des Neuronalen Netzwerks NN werden den Zellen Z (Neuronen) der Eingangsschicht N₀ zur gemeinsamen Bearbeitung wie dargestellt einzelne Pixel der Radiologie-Datensätze D1, D2, D3, D4 (welche hier den Teilmengen der Radiologie-Datensätze D1, D2, D3, D4 entsprechen) zugeordnet. Dabei enthält die Eingangsschicht N₀ 4·x·y Zellen Z (Neuronen) für die einzelnen Pixel der vier Radiologie-Datensätze D1, D2, D3, D4.

Nach Verarbeitung der Radiologie-Datensätze D1, D2, D3, D4 können die Ergebnisdaten ED an der Ausgangsschicht Nᵢ erfasst, z.B. von einer Ergebnisdateneinheit EE übernommen oder ausgelesen, werden. Diese Ergebnisdaten ED geben dann entsprechend der Art und des Trainings des Neuronalen Netzwerks NN beispielsweise an, ob eine Auffälligkeit in den Aufnahmen vorliegt oder nicht, ggf. welche Art von Auffälligkeit und/oder in welchem Umfang.

Figur 3 zeigt eine Darstellung eines besonders bevorzugten Ausführungsbeispiels zur Analyse der Radiologie-Daten D1, D2, D3, D4. Diese Figur ähnelt sehr Figur 2, zumindest was die Analyse betrifft. Im Unterschied zu Figur 2 werden hier jedoch die Radiologie-Datensätze D1, D2, D3, D4 anders gebildet.

In diesem Ausführungsbeispiel erfolgte nur eine Aufnahme bzw. Messung mit einem energieauflösenden bzw. "spektralen" Detektor, der die spektrale Energie der eintreffenden Photonen unterscheiden kann. Somit ergibt sich für Pixel dieser Bildaufnahme B ein Spektrum S von Bilddaten BD (die z.B. Grauwerten für eine Energie entsprechen können). Es ist klar, dass hierbei die verwendete Röntgenstrahlung ein entsprechend breites Energiespektrum aufweisen muss.

Die eintreffenden Signale am Detektor (Röntgenstrahlen) werden hier zunächst nach Schwellwerten in vier vorbestimmte Röntgenenergiespektren E1, E2, E3, E4 (die hier auch als "Spektralbereiche" bezeichnet werden können) klassifiziert und aus diesen Daten für einzelne Bilder als Radiologie-Datensätze D1, D2, D3, D4 ausgewählt. Die vier Radiologie-Datensätze D1, D2, D3, D4 könnten z.B. Daten zu vom Detektor 4 empfangenen Photonen mit einer Energie von 0 bis 40 KeV (erster Radiologie-Datensatz D1 basierend auf dem ersten Röntgenenergiespektrum bzw. Spektralbereich E1), 40 bis 65 KeV (zweiter Radiologie-Datensatz D2 basierend auf dem zweiten Röntgenenergiespektrum bzw. Spektralbereich E2), 65 bis 90 KeV (dritter Radiologie-Datensatz D3 basierend auf dem dritten Röntgenenergiespektrum bzw. Spektralbereich E3) und Energien größer als 90 KeV (vierter Radiologie-Datensatz D4 basierend auf dem vierten Röntgenenergiespektrum bzw. Spektralbereich E4) aufweisen.

Nach Verarbeitung der Radiologie-Datensätze D1, D2, D3, D4 können die Ergebnisdaten ED an der Ausgangsschicht Nᵢ erfasst werden, z.B. wieder mittels einer Ergebnisdateneinheit EE.

Ein konkretes klinisches Beispiel wäre ein Neuronales Netzwerk NN, welches Nierensteine charakterisieren kann. Eingangsdaten (Radiologie-Datensätze D1, D2, D3, D4) sind dann z.B. vier rekonstruierte (Bild)-Datensätze, die jeweils Messungen bei einem Röntgenenergiespektrum E1, E2, E3, E4 repräsentieren. Jedes Voxel jedes der Bilddatensätze würde dann einer Zelle Z der Eingangsschicht E₀ zugeordnet.

Die komplette Verarbeitungskette sieht dabei anlog zu dem Ausführungsbeispiel nach Figur 3 wie folgt aus:
Der Detektor "sortiert" zunächst die eintreffenden Rohdaten nach Röntgenenergiespektren E1, E2, E3, E4 während der Rotation (in seine "Bins"). Daraus entstehen dann als Rohdaten mehrere Sinogramme (nach Röntgenenergiespektren E1, E2, E3, E4). Daraus werden wiederum Radiologie-Datensätze D1, D2, D3, D4 rekonstruiert und diese in ein Neuronales Netz NN eingespeist.

Die Eingangsschicht E₀ könnte dabei der Anzahl und Struktur der Datensätze entsprechen, die Ausgangsschicht Nᵢ wäre eine Klassifikation "Vorhandensein eines Nierensteins" und "Urat vs. Calcium" (im Extremfall also nur einzelne Neuronen).

Dieses Neurale Netz NN würde vor dem ersten Analyseeinsatz zunächst ausreichend trainiert. Nach fertigem Training ist es dann in der Lage, Nierensteine zu erkennen und zu klassifizieren, wobei spektrale Informationen (Material des Nierensteins) genauso eingehen wie morphologische (Größe, Lage, Textur).

Zum Training des Neuronalen Netzes NN würden in diesem Beispiel Datensätze von Patienten P verwendet, bei denen das Vorhandensein die Zusammensetzung des Nierensteins bekannt ist, z.B. weil der Nierenstein gewonnen und chemisch analysiert wurde.

Nachfolgend wird noch ein möglicher Aufbau eines Röntgensystems 1 beschrieben, an dem das Verfahren einsetzbar ist. Bei den Erläuterungen wird davon ausgegangen, dass es sich bei dem Röntgenaufnahmesystem 1 um ein Computertomographiesystem 1 handelt. Grundsätzlich ist das Verfahren aber auch an anderen Röntgenaufnahmesystemen einsetzbar.

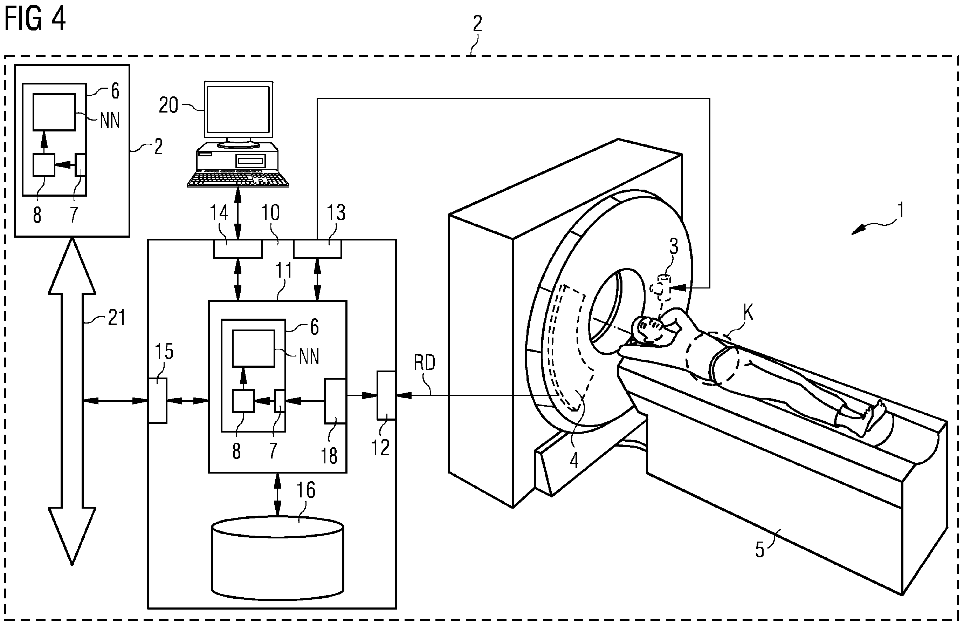

Figur 4 zeigt hierzu grob schematisch ein Computertomographiesystem 1 mit einer Steuereinrichtung 10 zur Durchführung des erfindungsgemäßen Verfahrens. Das Computertomographiesystem 1 weist in üblicher Weise einen Scanner mit einer Gantry auf, in der eine Röntgenquelle 3 rotiert, die jeweils einen Patienten durchstrahlt, welcher mittels einer Liege 5 in einen Messraum der Gantry hineingeschoben wird, so dass die Strahlung auf einen der Röntgenquelle 3 jeweils gegenüberliegenden Detektor 4 trifft. Es wird ausdrücklich darauf hingewiesen, dass es sich bei dem Ausführungsbeispiel gemäß Figur 4 nur um ein Beispiel eines CTs handelt und die Erfindung auch an beliebigen CT-Konstruktionen, beispielsweise mit ringförmigem feststehendem Röntgendetektor und/oder mehreren Röntgenquellen genutzt werden kann.

Ebenso sind bei der Steuereinrichtung 10 nur die Komponenten dargestellt, die für die Erläuterung der Erfindung wesentlich oder hilfreich sind. Grundsätzlich sind derartige CT-Systeme und zugehörige Steuereinrichtungen dem Fachmann bekannt und brauchen daher nicht im Detail erläutert zu werden.

In diesem Beispiel sind die Röntgenquelle 3 und der Detektor 4 zu einem Aufnahmeverfahren ausgestaltet, welches mehrere Aufnahmeenergien nutzt. Beispielsweise kann der Detektor 4 mehr als nur einen Signalwert pro Pixel zu erzeugen, und dabei die spektrale Energie der eintreffenden Photonen unterscheiden. Es könnten in einem anderen Ausführungsbeispiel auch zwei oder mehr Röntgenquellen 3 und Detektoren 4 vorliegen, die für Aufnahmen bei jeweils unterschiedlichen Energien ausgelegt sind.

Eine Kernkomponente der Steuereinrichtung 10 ist hier ein Prozessor 11, auf dem verschiedene Komponenten in Form von Softwaremodulen realisiert sind. Die Steuereinrichtung 10 weist weiterhin eine Terminalschnittstelle 14 auf, an die ein Terminal 20 angeschlossen ist, über das ein Bediener die Steuereinrichtung 10 und somit das Computertomographiesystem 1 bedienen kann. Eine weitere Schnittstelle 15 ist eine Netzwerkschnittstelle zum Anschluss an einen Datenbus 21, um so eine Verbindung zu einem RIS bzw. PACS herzustellen. Über diesen Bus 21 können beispielsweise Radiologie-Daten D1, D2, D3, D4 zu einer Befundungsstation 2 gesendet werden.

Über eine Steuerschnittstelle 13 kann von der Steuereinrichtung 10 der Scanner angesteuert werden, d. h. es werden z.B. die Rotationsgeschwindigkeit der Gantry, die Verschiebung der Patientenliege 5 und die Röntgenquelle 3 selbst gesteuert. Über eine Akquisitionsschnittstelle 12 werden die Rohdaten RD aus dem Detektor 4 ausgelesen. Weiterhin weist die Steuereinrichtung 10 eine Speichereinheit 16 auf, in der z.B. verschiedene Messprotokolle hinterlegt sind.

Als eine Softwarekomponente ist auf dem Prozessor 11 eine Analyseeinheit 6 implementiert. Diese Analyseeinheit 6 erhält über eine Datenschnittstelle 7 in diesem Beispiel rekonstruierte Bilddaten BD von einer eine Bilddaten-Rekonstruktionseinheit 18, mit welcher aus den über die Datenakquisitions-Schnittstelle 12 erhaltenen Rohdaten RD die gewünschten Bilddaten BD rekonstruiert werden. Diese Bilddaten-Rekonstruktionseinheit 18 kann aber in anderen Anwendungsformen auch weggelassen werden und bei der Analyse direkt mit den Rohdaten RD gearbeitet werden.

Die Analyseeinheit 6 umfasst des Weiteren eine Aufteilungseinheit 8 welche dazu ausgelegt ist, nach einer Erfassung von spektrale Rohdaten RD oder in diesem Beispiel rekonstruierten Bilddaten BD basierend auf Röntgendaten aus zumindest zwei Messungen mit unterschiedlichen Röntgenenergiespektren E1, E2, E3, E4 durch die Datenschnittstelle 7 die einzelnen Radiologie-Datensätze D1, D2, D3, D4 durch Aufteilung der Rohdaten RD oder der Bilddaten BD entsprechend ihrer Röntgenenergiespektren E1, E2, E3, E4 zu erstellen. Eine Aufteilung der Daten in Radiologie-Datensätze D1, D2, D3, D4 wurde oben bei der Beschreibung zu den Figuren 2 und 3 genauer dargestellt.

Die Analyseeinheit 6 umfasst zudem ein für eine Analyse von Radiologie-Datensätzen D1, D2, D3, D4 ausgebildetes, insbesondere bereits trainiertes, Neuronales Netzwerk NN, welches eine Eingangsschicht N0 mit mehreren Zellen Z, eine Anzahl von Zwischenschichten N1 und eine Ausgangsschicht Ni umfasst, welche ein radiologisches Ergebnis repräsentiert (s. dazu Figuren 2 und 3). Die Analyseeinheit ist dabei dazu ausgelegt, mittels des Neuronalen Netzwerks NN die bereitgestellten Radiologie-Daten zu analysieren, wie weiter oben bereits ausführlicher dargestellt wurde.

Die Befundungsstation 2 ist sehr ähnlich zu der Steuereinrichtung 10 aufgebaut. Auch sie umfasst die vorbeschriebene Analyseeinheit 6. Die Befundungsstation 2 und die Steuereinrichtung 10 können wie hier dargestellt gemeinsam vorliegen (in dem beide eine erfindungsgemäße Analyseeinheit 6 umfassen), Das medizintechnische bildgebende System kann aber auch nur in einer dieser beiden Komponenten eine erfindungsgemäße Analyseeinheit 6 aufweisen.

Es wird abschließend noch einmal darauf hingewiesen, dass es sich bei den vorhergehend detailliert beschriebenen Analyse-Verfahren sowie bei den dargestellten Vorrichtungen bzw. Einheiten bzw. dem Computertomographiesystem 1 lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Modul" nicht aus, dass die betreffenden Komponenten aus mehreren zusammenwirkenden Teil-Komponenten bestehen, die gegebenenfalls auch räumlich verteilt sein können.

## Patentansprüche

1. Analyse-Verfahren zur automatischen Ermittlung radiologischer Ergebnisdaten von Radiologie-Datensätzen (D1, D2, D3, D4), umfassend die Schritte:
- Bereitstellung eines ersten Radiologie-Datensatzes (D1) zumindest basierend auf Röntgendaten eines ersten Röntgenenergiespektrums (E1),
- Bereitstellung zumindest eines zweiten Radiologie-Datensatzes (D2, D3, D4) zumindest basierend auf Röntgendaten eines zweiten Röntgenenergiespektrums (E2, E3, E4),
- Bereitstellung einer Analyseeinheit (6) umfassend ein zu einer Analyse von Radiologie-Datensätzen (D1, D2, D3, D4) ausgebildetes Neuronales Netzwerk (NN), welches eine Eingangsschicht (N₀) mit mehreren Zellen (Z), eine Anzahl von Zwischenschichten (N₁) und eine Ausgangsschicht (Nᵢ) umfasst, welche ein radiologisches Ergebnis repräsentiert,
- Analyse des ersten Radiologie-Datensatzes (D1) und zumindest des zweiten Radiologie-Datensatzes (D2, D3, D4) mittels des Neuronalen Netzwerks (NN), wobei zumindest Teilmengen des ersten Radiologie-Datensatzes (D1) sowie des zweiten Radiologie-Datensatzes (D2, D3, D4) unterschiedlichen Zellen der Eingangsschicht (N₀) zur gemeinsamen Bearbeitung im Neuronalen Netzwerk (NN) zugeordnet werden,
- Erfassung von Ergebnisdaten an der Ausgangsschicht.

2. Analyse-Verfahren nach Anspruch 1, wobei einer Anzahl von Zellen (Z) der Eingangsschicht (N₀) jeweils genau ein Bildpunktwert oder Rohdatenwert des ersten Radiologie-Datensatzes (D1) oder des zweiten Radiologie-Datensatzes (D2, D3, D4) zugeordnet wird,
wobei bevorzugt jeder einzelne Bildpunktwert und/oder jeder einzelne Rohdatenwert der Radiologie-Datensätze (D1, D2, D3, D4) einer Zelle (Z) der Eingangsschicht (N₀) zugeordnet ist.

3. Analyse-Verfahren nach einem der vorangehenden Ansprüche, wobei in einem Analyseschritt nur ein Teil der zu analysierenden Radiologie-Datensätze (D1, D2, D3, D4) analysiert wird und ein anderer Teil der zu analysierenden Radiologie-Datensätze (D1, D2, D3, D4) in mindestens einem weiteren Analyseschritt analysiert wird.

4. Analyse-Verfahren nach einem der vorangehenden Ansprüche, wobei das Neuronale Netzwerk (NN) mindestens 2, bevorzugt mindestens 5, besonders bevorzugt mindestens 20, Zwischenschichten (N1) umfasst.

5. Analyse-Verfahren nach einem der vorangehenden Ansprüche, wobei die Analyseeinheit (6) als Neuronales Netzwerk (NN) ein Faltungsnetz oder ein Long-Term-Short-Term Memory-Netz umfasst.

6. Analyse-Verfahren nach einem der vorangehenden Ansprüche, wobei zumindest einer der bereitgestellten Radiologie-Datensätze (D1, D2, D3, D4), zumindest auf Röntgendaten zweier unterschiedlicher Röntgenenergiespektren (E1, E2, E3, E4) beruht, vorzugsweise auf Kombinationsdaten von Radiologie-Datensätzen (D1, D2, D3, D4) basierend auf Röntgendaten der unterschiedlichen Röntgenenergiespektren (E1, E2, E3, E4), insbesondere in Form einer Summe und/oder einer Differenz und/oder eines Quotienten und/oder eines Produkts, insbesondere einer Kombination dieser Rechenoperationen.

7. Analyse-Verfahren nach einem der vorangehenden Ansprüche, wobei zur Bereitstellung des ersten Radiologie-Datensatzes (D1) und des zweiten Radiologie-Datensatzes (D2, D3, D4) ein Gesamt-Röntgen-Datensatz aufgeteilt wird, der mit einem dritten Röntgenenergiespektrum gemessen wurde, welches das erste Röntgenenergiespektrum (E1) und das zweite Röntgenenergiespektrum (E2, E3, E4) umfasst,
vorzugsweise durch Aufteilung von Rohdaten (RD) einer Messung mit dem dritten Röntgenenergiespektrum in unterschiedliche Rohdatensätze und einer Rekonstruktion von unterschiedlichen Bilddatensätzen auf Basis der unterschiedliche Rohdatensätze.

8. Analyseeinheit (6) zur automatischen Ermittlung radiologischer Ergebnisdaten von Radiologie-Datensätzen (D1, D2, D3, D4), umfassend:
- eine Datenschnittstelle (7) zur Erfassung
i) eines ersten Radiologie-Datensatzes (D1) zumindest basierend auf Röntgendaten eines ersten Röntgenenergiespektrums (E1), und
ii) zumindest eines zweiten Radiologie-Datensatzes (D2, D3, D4) zumindest basierend auf Röntgendaten eines zweiten Röntgenenergiespektrums (E2, E3, E4),
- ein zu einer Analyse von Radiologie-Datensätzen (D1, D2, D3, D4) ausgebildetes Neuronales Netzwerk (NN), welches eine Eingangsschicht (N₀) mit mehreren Zellen (Z), eine Anzahl von Zwischenschichten (N₁) und eine Ausgangsschicht (Nᵢ) umfasst, welche ein radiologisches Ergebnis repräsentiert,
wobei die Analyseeinheit zu einer Analyse des ersten Radiologie-Datensatzes (D1) und zumindest des zweiten Radiologie-Datensatzes (D2, D3, D4) mittels des Neuronalen Netzwerks (NN) ausgelegt ist, wobei zumindest Teilmengen des ersten Radiologie-Datensatzes (D1) sowie des zweiten Radiologie-Datensatzes (D2, D3, D4) unterschiedlichen Zellen der Eingangsschicht (N₀) zur gemeinsamen Bearbeitung im Neuronalen Netzwerk (NN) zugeordnet werden, und
wobei die Analyseeinheit zu einer Erfassung von Ergebnisdaten an der Ausgangsschicht ausgelegt ist.

9. Neuronales Netzwerk, insbesondere für eine Analyseeinheit (6) nach Anspruch 8, umfassend eine Eingangsschicht (N₀) mit mehreren Zellen (Z), eine Anzahl von Zwischenschichten (N₁) und eine Ausgangsschicht (Nᵢ), welche ein radiologisches Ergebnis repräsentiert, welches dazu ausgelegt ist, dass unterschiedlichen Zellen der Eingangsschicht (N₀) zur gemeinsamen Bearbeitung zumindest Teilmengen eines ersten Radiologie-Datensatzes (D1) basierend zumindest auf Röntgendaten eines ersten Röntgenenergiespektrums (E1) sowie zumindest eines zweiten Radiologie-Datensatzes (D2, D3, D4) zumindest basierend auf Röntgendaten eines zweiten Röntgenenergiespektrums (E2, E3, E4) zugeordnet werden,
und das zusätzlich dazu ausgelegt ist, radiologische Ergebnisdaten zu generieren und an der Ausgangsschicht (Nᵢ) zur Verfügung zu stellen.

10. Steuereinrichtung (10) zur Steuerung eines Röntgenaufnahmesystems (1), insbesondere eines Computertomographiesystems (1), welche eine Analyseeinheit nach Anspruch 8 umfasst und/oder zur Durchführung eines Analyse-Verfahrens nach einem der Ansprüche 1 bis 7 ausgestaltet ist.

11. Befundungsstation (2) zur Kopplung mit einem Röntgenaufnahmesystem (1), insbesondere mit einem Computertomographiesystem (1), welche Befundungsstation (2) eine Analyseeinheit nach Anspruch 8 umfasst und/oder zur Durchführung eines Analyse-Verfahrens nach einem der Ansprüche 1 bis 7 ausgestaltet ist.

12. Medizintechnisches bildgebendes System (9) umfassend ein Röntgenaufnahmesystem (1) und eine Analyseeinheit (6) nach Anspruch 8 bevorzugt in Form einer Befundungsstation nach Anspruch 12 und/oder einer Steuereinrichtung (10) zur Steuerung des Röntgenaufnahmesystems (1) nach Anspruch 11.

13. Medizintechnisches bildgebendes System nach Anspruch 12, wobei das Röntgenaufnahmesystem (1) einen energieauflösenden Detektor, besonders bevorzugt einen direkt konvertierenden Detektor, aufweist.

14. Computerprogrammprodukt mit einem Computerprogramm, welches direkt in eine Speichereinrichtung eines Rechensystems oder einer Steuereinrichtung (10) eines medizintechnischen bildgebenden Systems (1) ladbar ist, mit Programmabschnitten, um alle Schritte des Analyse-Verfahrens nach einem der Ansprüche 1 bis 7 auszuführen, wenn das Computerprogramm in dem Rechensystem oder der Steuereinrichtung (10) ausgeführt wird.

15. Computerlesbares Medium, auf welchem von einer Rechnereinheit einlesbare und ausführbare Programmabschnitte gespeichert sind, um alle Schritte eines Analyse-Verfahrens nach einem der Ansprüche 1 bis 7 auszuführen, wenn die Programmabschnitte von der Rechnereinheit ausgeführt werden.
